# EUROPEAN PATENT APPLICATION

(11) **EP 2 060 293 A1**
(43) Date of publication of application: **20.05.2009**
(21) Application number: 08168770.9
(22) Date of filing: 10.11.2008
(51) Int. Cl.: A61M 16/04, A61M 25/10, G01L 7/06

(54) **Indicator for cuffed medical device**

(30) Priority: 14.11.2007 JP 2007295706
(71) Applicant: Advanex Inc., Tokyo 114-8581 (JP); Wonderworks Corporation, Kita-ku Tokyo (JP)
(72) Inventor: Chikashige, Kiyoshi, Tokyo (JP)
(74) Representative: Ilgart, Jean-Christophe

(57) **Abstract**

An indicator for a cuffed medical device, attached to a cuffed medical device to indicate internal pressure in a cuff of the medical device. The indicator includes: an indicator main body 2 which includes a check valve 10 adapted to be connected to an injection means for injecting a fluid into the cuff, a connecting section 3 formed integrally with the indicator main body 2 for connecting to the cuffed medical device, the connecting section communicating with the inside of the cuff, and a communications path 9 for making the check valve 10 and the connecting section 3 communicate with each other; and a balloon 5 provided in the indicator main body 2. The inside of the balloon 5 is made to communicate with the communication path 9 and is inflatable and deflatable in accordance with the internal pressure in the cuff. The balloon 5 includes an index body 19 which is moved forward and backward in accordance with the degree of inflation and deflation of the balloon 5 in order to make the degree of inflation and deflation of the balloon 5 visually recognizable. With the indicator for a cuffed medical device according to the invention, reduction in internal pressure can be readily and precisely determined.

## Description

### BACKGROUND OF THE INVENTION

### Field of the Invention

The present invention relates to an indicator attached to a cuffed medical device used to indicate internal pressure in a cuff of the medical device.

This application claims priority of Japanese Patent Application No. 2007-295706, filed on November 14, 2007, the disclosure of which is incorporated herein by reference in its entity.

### Description of the Related Art

A process of blocking a bodily passage with a cuffed (ballooned) medical device has been well known in the medical field. A tracheal tube, for example, is used for artificial respiration. One distal end of the tracheal tube is inserted through the mouth (i.e., orally) or the nose (i.e., nasally) of a patient. The other distal end is connected to an artificial respirator. The cuff of the tracheal tube is inflated to block the trachea at a position above the organs (i.e., the lungs) to allow the patient to forcibly breathe by the artificial respirator which is in communication with the inside of the tracheal tube.

Another example includes a balloon, catheter for pulmonary artery. The balloon catheter is inserted in a blood vessel via suitable blood vessels such as a carotid artery, and the catheter is carried to the pulmonary artery on the blood flow.

The balloon is then inflated within the pulmonary artery so as to measure the "wedge pressure" at which the blood enters the lungs using a sensor provided at a distal end of a catheter.

In the conventional cuffed (ballooned) medical device adapted to block a bodily passage with an inflated cuff, however, problems may arise from overinflation of the cuff. An overinflated cuff may put pressure on and cause damage to trachea mucous in the tracheal tube, and may cause damage to the blood vessels in the balloon catheter for pulmonary artery.

To address such problems, a cuff (balloon) inflation support tool has been provided which prevents overinflation of a cuff during blocking of a bodily passage so as to simplify the procedure, and thus can be applied to various cuffed (ballooned) medical devices (see Japanese Unexamined Patent Application Publication (JP-A) No. 2003-116999).

In the cuffed (ballooned) medical device, overinflation of the cuff may cause the above-described problems whereas reduced internal pressure in the cuff may also cause problems. When the internal pressure in the cuff is reduced and the cuff no longer maintains a predetermined degree of inflation, the trachea may be blocked, resulting in insufficient forced respiration with an artificial respirator. A function of the cuff as a float during carrying the catheter to the pulmonary artery via blood flow also becomes insufficient. This is because the cuff is made of rubber or another material, and thus the flowing air escapes gradually from the cuff in a molecular level to reduce the internal pressure and thus the degree of inflation.

A user has conventionally detected reduction in internal pressure by recognizing visually or tactually a degree of inflation of a pilot balloon provided at a branch section where an injector for injecting air into the cuff of the cuffed medical device is connected.

However, in this method of detecting the reduction in the internal pressure in the cuff by checking the degree of inflation of the pilot balloon visually or tactually takes time and effort and also requires a certain amount of experience. In particular, the softness of human earlobe is considered a suitable degree of inflation for the tactual check of the pilot balloon. Actually, however, the definition of the degree is ambiguous and thus it is significantly difficult to determine the reduction in internal pressure in the cuff readily and precisely.

In view of the aforementioned, an object of the invention is to provide an indicator for a cuffed medical device adapted to readily and precisely detect a reduction in internal pressure in a cuff.

### SUMMARY OF THE INVENTION

In order to achieve the aforementioned object, an indicator for a cuffed medical device according to the invention is an indicator for a cuffed medical device, attached to a cuffed medical device to indicate the internal pressure in a cuff of the medical device, the indicator including: an indicator main body which includes a check valve adapted to be connected to an injection mechanism such as an injector for injecting a fluid into the cuff, a connecting section formed integrally with the indicator main body for connecting to the cuffed medical device, the connecting section communicating with the inside of the cuff, and a communication path for making the check valve and the connecting section communicate with each other; and a balloon provided in the indicator main body, wherein the inside of the balloon is made to communicate with the communication path and is inflatable and deflatable in accordance with the internal pressure in the cuff; and the balloon includes an index body which is moved forward and backward in accordance with the degree of inflation and deflation of the balloon in order to make the degree of inflation and deflation of the balloon visually recognizable.

In the indicator for a cuffed medical device, an injection mechanism such as an injector is connected to a check valve. A fluid such as air is introduced into the cuff through the communication path and the connecting section of the indicator main body. Thus, the cuff can be inflated to a predetermined degree. The balloon communicates with the communication path, and the fluid can also be introduced into the balloon. Even if the injection mechanism is removed from the check valve in this state, the check valve prevents air (fluid) in the cuff from escaping from the balloon.

When the cuff is kept in its inflated state, the balloon communicating with the cuff via the communication path inflates in accordance with the internal pressure in the cuff. If the balloon is exposed to the outside of the indicator main body, the user can detect the internal pressure in the cuff by touching the exposed portion of the balloon with fingers and checking the degree of inflation (the degree of inflation and deflation).

The index body moves forward and backward in accordance with the degree of inflation and deflation of the balloon. This configuration allows the user to readily and precisely check reduction in internal pressure in the cuff through visual recognition of the index body.

The connecting section to be connected to the cuffed medical device is formed integrally with the indicator main body. With this configuration, a problem of the connecting section being removed from the indicator main body during operation of the cuffed medical device is avoided.

In the indicator for a cuffed medical device, it is preferred that the balloon includes a bellow-shaped expandable section in which the index body is provided, wherein the bellow-shaped expandable section is adapted to expand and contract so as to move the index body forward and backward.

With this configuration, the bellow-shaped expandable section of the balloon at which the index body is provided expands and contracts mechanically to move the index body forward and backward. Compared with a case in which the expandable section expands and contracts due only to elastic deformation of a material made of elastic body, the balloon according to the invention suffers less from deterioration such as fatigue and restorability is maintained over time. In this manner, the relationship between the internal pressure in the cuff and the forward and backward movement of the index body is kept constant over time.

In the indicator for a cuffed medical device, it is preferred that the balloon is accommodated in the indicator main body, and the indicator main body has an opening formed thereon through which an inflating/deflating surface of the balloon is exposed.

This configuration allows the user to detect the internal pressure in the cuff by touching the inflating/deflating surface of the balloon with fingers and check the internal pressure in the cuff.

The balloon is accommodated in and thus protected by the indicator main body. The balloon can be protected from being damaged by, for example, unexpected impacts, whereby the indicator for a cuffed medical device can be handled easily.

In the indicator for a cuffed medical device of the invention, when the cuff is kept inflated, the balloon communicating with the cuff inflates in accordance with the internal pressure in the cuff. The user can detect the internal pressure in the cuff by touching the balloon with fingers in order to check the degree of inflation (the degree of inflation and deflation) and detect the internal pressure in the cuff. The user can also visually recognize the index body to readily and precisely detect reduction in internal pressure in the cuff. Thus, the user can determine whether or not the internal pressure in the cuff is suitable more reliably in the check of the internal pressure in the cuff by touching the cuff and visually recognizing the index body.

Since the connecting section is formed integrally with the indicator main body, the connecting section cannot be separated from the indicator main body, and thus operability of the cuffed medical device can be improved.

### BRIEF DESCRIPTION OF THE DRAWINGS

FIG 1A is a schematic view of a cuffed medical device to which an indicator according to the invention is attached.
FIG. 1B is a schematic view of a conventional cuffed medical device.

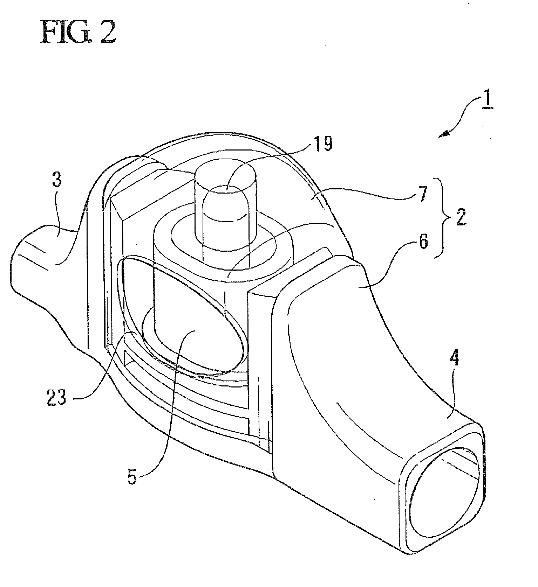

FIG. 2 is a schematic perspective view showing the structure of an embodiment of an indicator for a cuffed medical device according to the present invention.
FIG. 3A is a schematic cross-sectional side view showing the structure of the indicator for the cuffed medical device shown in FIG. 2.
FIG. 3B is a cross-sectional view taken along the line A-A in FIG. 3A.
FIG. 4 is an exploded perspective view of the indicator for the cuffed medical device shown in FIG. 2.
FIG. 5 is a perspective view showing the structure of a valve element.
FIGS. 6A to 6C illustrate operation of an index body.
FIG. 7 illustrates the cuffed medical device upon usage.

### DESCRIPTION OF THE PREFERRED EMBODIMENTS

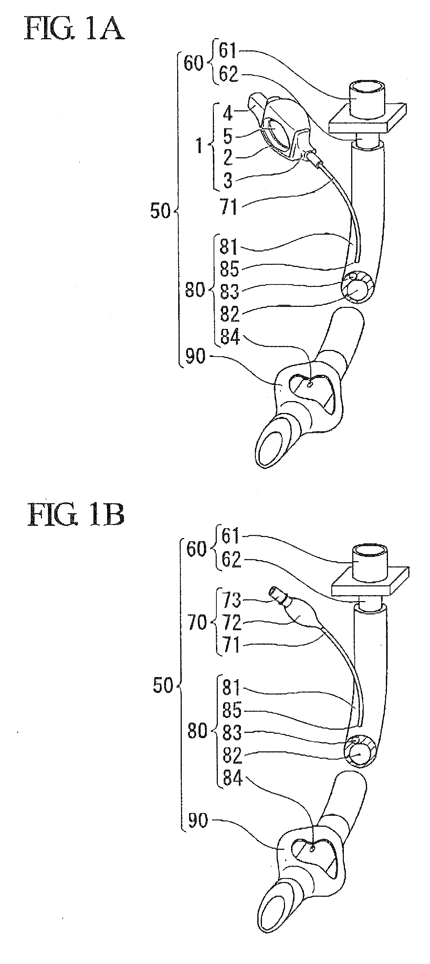

Hereinafter, the invention will be described in detail. First, a description, will be given on a cuffed medical device to which an indicator is attached. FIG. 1A shows a cuffed medical device with an indicator 1 according to the present invention. FIG. 1B shows a conventional cuffed medical device.

In FIG. 1B, a conventional ordinary tracheal tube denoted generally as 50 is shown as a cuffed medical device. The cuffed medical device (tracheal tube) 50 includes a connector 60 to be connected to an artificial respirator, an injecting portion 70 used for inflating the cuff, a tube 80 inserted in a patient's trachea, and a cuff (balloon) 90 for blocking the trachea.

The connector 60, formed as a cylinder, includes a first connecting tube 61 to be connected to an artificial respirator, and a second connecting tube 62 made to fit air-tightly into a later-described tube main body 81.

The injecting portion 70 includes a connecting tube 71 connected to the tube 80, a pilot balloon 72 and a check valve 73. The pilot balloon 72 is formed as a balloon with openings at both ends. A user can check the internal pressure in the cuff communicating with the pilot balloon 72 through visual and tactile recognition of the inflated state of the pilot balloon 72. The check valve 73 is adapted to allow air (fluid) to flow thereinto only from the end opposite to the end of the pilot balloon 72, and air is prevented from escaping from the check valve 73 from the pilot balloon 72 side. The check valve 73 is connected to an injector to serve as an air inlet.

With the thus-configured injecting portion 70, although the internal pressure in the cuff 90 is to be determined (i.e., recognized) by the user viewing or touching the pilot balloon 72, visual recognition of the degree of inflation as described above requires time and effort, or it requires a certain amount of experience.

In the present invention, as shown in FIG. 1A, an indicator 1 is connected to a connecting tube 71. The indicator 1 includes an indicator main body 2, a cuff side connecting section 3 and an injector side connecting section 4 formed integrally with the indicator main body 2, and a balloon 5 accommodated in the indicator main body 2. The cuff side connecting section 3 is connected to the connecting tube 71. With the indicator 1, a user can check the internal pressure in the cuff 90 by pinching (touching) the balloon 5 with fingers or by visually recognizing an index body (not shown) of the balloon 5. The indicator 1 will be described in detail later.

The tube 80 includes a tube main body 81, a first hole 82 which penetrates the entire length of the tube main body 81, a second hole 83 which penetrates a part of the tube main body 81, a first opening 84 for making the second hole 83 communicate with external air at a patient side distal end of the tube main body 81, and a second opening 85 for making the second hole 83 communicate with external air at another distal end of the tube main body 81. At the second opening 85, the connecting tube 71 is joined and fixed air-tight to the tube main body 81.

The cuff 90 is formed as a balloon disposed to cover the first opening 84. The cuff 90 is airtightly joined and fixed to the external surface of the tube main body 81 at both ends thereof. The inside of the cuff is made to communicate with external air only through the first opening 84. This means that the cuff 90 communicates with the second hole 83 and the connecting tube 71 via the first opening 84.

Next, the indicator 1 will be described in detail. The indicator 1 is an embodiment of an indicator for a cuffed medical device according to the present invention. As shown in FTG. 2, the indicator 1 includes the indicator main body 2, the cuff side connecting section 3 and the injector side connecting section 4 formed integrally with the indicator main body 2, and the balloon 5 accommodated in the indicator main body 2.

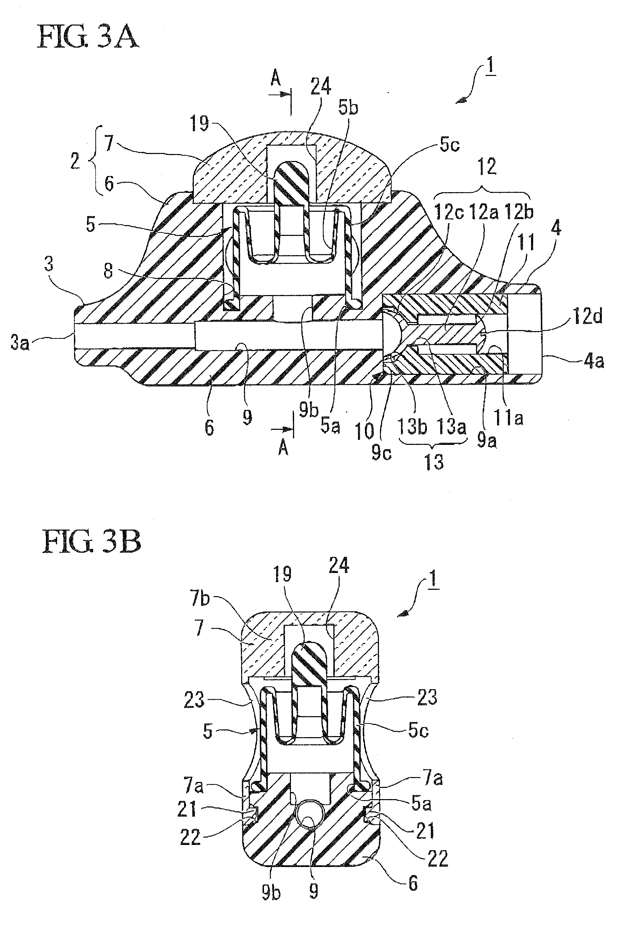

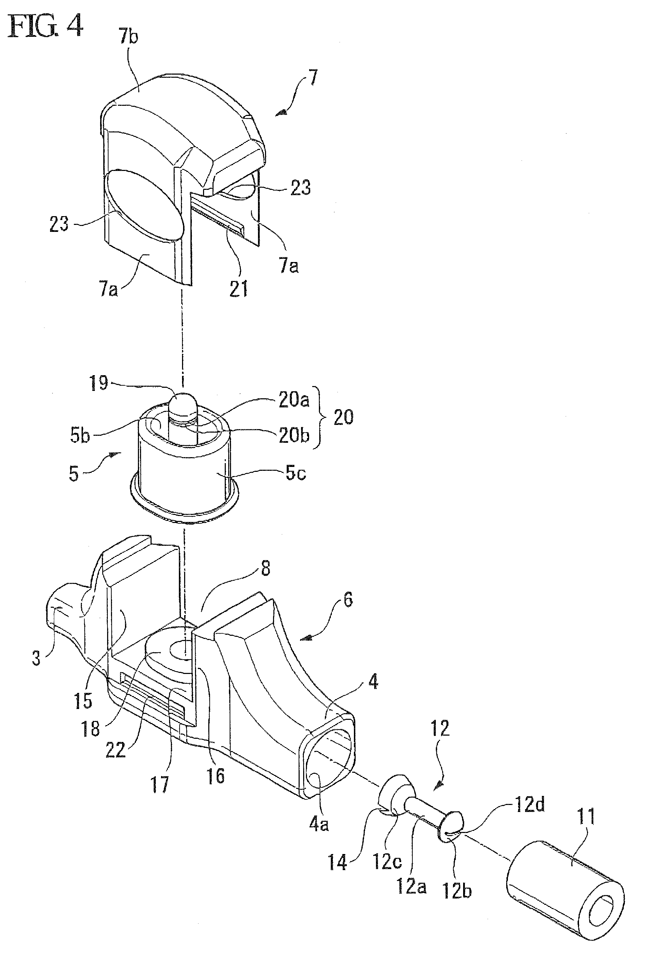

As shown in a cross-sectional side view in FIG. 3A and an exploded perspective view in FIG 4, the indicator main body 2 is constituted by a main body 6 and a cap 7 fit into the main body 6. The indicator main body 2 is made of comparatively hard transparent resin such as polycarbonate. The cuff side connecting section 3 is formed at one end, and the injector side connecting section 4 is formed at the other end of the main body 6. A balloon accommodating section 8 is formed between the cuff side connecting section 3 and the injector side connecting section 4. The main body 6 includes a through hole 9 below the balloon accommodating section 8 for making the cuff side connecting section 3 and the injector side connecting section 4 communicate with each other. The through hole 9 provides a communication path (hereinafter, the communication path is also denoted as 9). As shown in FIG. 3A, the communication path 9 has an enlarged diameter section 9a having a larger inner diameter provided at the side of the injector side connecting section 4.

The cuff side connecting section 3 has an opening 3a at one end thereof, which is formed as one of the openings of the communication path (through hole) 9. As shown in FIGS. 3A and 4, the injector side connecting section 4 also has an opening 4a at an end thereof, which is formed as the other of the openings of the communication path 9. With this configuration, the injector side connecting section 4 and the cuff side connecting section 3 are made to communicate with each other through the communication path 9 in the indicator main body 2. Air (fluid) introduced from the injector side connecting section 4 flows toward the cuff side connecting section 3 as described later.

The communication path 9 includes an upwardly branched branch section 9b in the substantially middle section thereof. The branch section 9b forms a part of the communication path 9, and has an opening at the side of the balloon accommodating section 8 so as to communicate with the inside of the balloon 5 as described later.

The cuff side connecting section 3 is joined and fixed to the connecting tube 71 of the cuffed medical device 50 with the connecting tube 71 inserted and if necessary is bonded or welded to the cuff side connecting section 3 at the opening 3 a.

The injector side connecting section 4 is connected to the injection mechanism such as an injector (not shown) with the injection mechanism inserted in the opening 4a. Thus, the injector side connecting section 4 has a check valve 10 in the communication path (through hole) 9 at the opening 4a side.

The check valve 10 is constituted by a resin-made cylindrical sleeve 11 provided in the enlarged diameter section 9a of the communication path 9 and a valve element 12 provided in the sleeve 11. The sleeve 11 is press-fit in the enlarged diameter section 9a such that an inner end surface of the sleeve 11 is in contact with a step portion 9c located between the enlarged diameter section 9a and the through hole 9 at the branch section 9b side. In this state, the sleeve 11 is fixed in the enlarged diameter section 9a. The sleeve 11 has a valve seat 13 provided in an inner hole 11a at the branch section 9b side. The valve seat 13 protrudes into the inner hole 11a. The valve seat 13 includes a small hole section 13a formed at the opening 4a side and a tapered portion 13b formed at the branch section 9b side. With this configuration, the valve seat 13 can hold the valve element 12 adapted to be movable in forward and backward directions. At the tapered portion 13b of the valve seat 13, a tapered surface is provided having a diameter increasing gradually from the small hole section 13a toward outside.

The valve element 12 includes a cylindrical axis 12a, a substantially semi-sphere shaped contact portion 12b formed at one distal end of the axis 12a, and a bowl-shaped expandable section 12c formed at the other distal end of the axis 12a. The valve element 12 is made of elastic resin such as vinyl chloride or elastic rubber. The axis 12a of the valve element 12 is movably inserted in the small hole section 13a of the valve seat 13 with the contact portion 12b facing with the opening 4a. The contact portion 12b includes a groove 12d provided on the surface at the opening 4a side, i.e., on the spherical surface. With this configuration, an inlet of the injector is brought into contact with the spherical surface of the contact portion 12b. At this time, the inlet of the groove 12d is not blocked by the contact portion 12b and the inside of the inlet is made to communicate with the inner hole 11a of the sleeve 11 via the groove 12d.

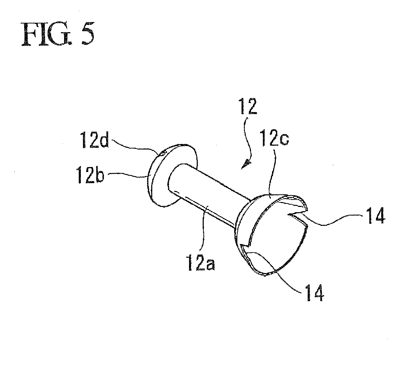

The expandable section 12c is bowl-shaped as described above and is thus elastically deformable. The expandable section 12c is disposed such that the open end of the bowl, i.e., the end of the valve element 12, faces with the branch section 9b, and an outer surface of the bottom of the bowl-shaped expandable section 12c is in contact with the tapered surface of the tapered portion 13b of the valve seat 13. The open end of the bowl-shaped expandable section 12c is in contact with the step portion 9c. With this configuration, the expandable section 12c is held in the enlarged diameter section 9a (i.e., in the communication path 9) between the tapered portion 13b of the valve seat 13 and the step portion 9c within the sleeve 11. As shown in FIG. 5, a plurality of (two in the present embodiment) V-shaped cutouts 14 are formed at an edge portion of the expandable section 12c at the open end of the bowl-shaped expandable section 12c. The V-shaped cutouts 14 of the expandable section 12c allow the small hole section 13a of the valve seat 13 and the branch section 9b side of the communication path 9 to communicate with each other even when the open end side of the bowl-shaped expandable section 12c is in contact with the step portion 9c.

As shown in FIG. 3A, the elastically deformable expandable section 12c abuts the step portion 9c and receives reaction force from the step portion 9c so as to urge the axis 12a toward the opening 4a. In the normal state of the valve element 12, the outer surface of the bottom of the bowl-shaped elastic portion 12c is in airtight contact with the tapered surface of the tapered portion 13b of the valve seat 13 due to urging force af the elastic portion 12c as described above, thereby airtightly blocking the small hole section 13a.

Since the valve element 12 is made of elastic resin or elastic rubber, the contact portion 12b of the valve element 12 can be fit into the small hole section 13a of the valve seat 13 and therefore the valve element 12 can be easily attached to the valve seat 13 due to elastic deformation of the contact portion 12b.

The check valve 10 according to the invention includes the injector side connecting section 4 in which an injection mechanism is inserted, the sleeve 11 disposed in the communication path (through hole) 9, the valve seat 13 provided in the sleeve 11 and the valve element 12 held on the valve seat 13.

As shown in FIG. 4, the balloon accommodating section 8 is formed between a cuff side wall 15 formed inside of the cuff side connecting section 3 and an injector side wall 16 formed inside of the injector side connecting section 4. A balloon placement surface 17 is provided between the cuff side wall 15 and the injector side wall 16. The space above the balloon placement surface 17 serves as a balloon accommodating section 8. A protruding portion 18 which is substantially elliptically-shaped (or substantially oval) in planar view is formed in the center of the balloon placement surface 17. An opening at the center of the protruding portion 18 is connected to the branch section 9b.

The balloon 5 is formed as a bag made of elastic resin such as silicon or vinyl chloride. The entire balloon 5 is inflated or deflated due to elastic deformation of the elastic resin, i.e., the balloon 5 is inflated when air is introduced thereinto and deflated as air is released. As shown in FIGS. 3A and 3B, the balloon 5 is airtightly fixed to the balloon placement surface 17 while a flanged opening 5a is fit on, engaged with and bonded or welded to the protruding portion 18 of the balloon placement surface 17. With this configuration, the inside of the balloon 5 is made to communicate with the communication path 9 via the branch section 9b.

The balloon 5 includes a bellow-shaped expandable section 5b at the side opposite to the opening 5a. The expandable section 5b has the following configuration: an upper portion (opposite to the opening 5a) of the side cylinder portion 5c extending from the opening 5a is folded inward; and the central portion of the folded section of the side cylinder portion 5c is again folded upward. With this configuration, when air (fluid) is introduced into the balloon 5, the balloon 5 is inflated in accordance with the amount of air (fluid). At this time, the bellow-shaped expandable section 5b is mechanically expanded as the balloon 5 becomes inflated and is mechanically contracted as the balloon 5 becomes deflated.

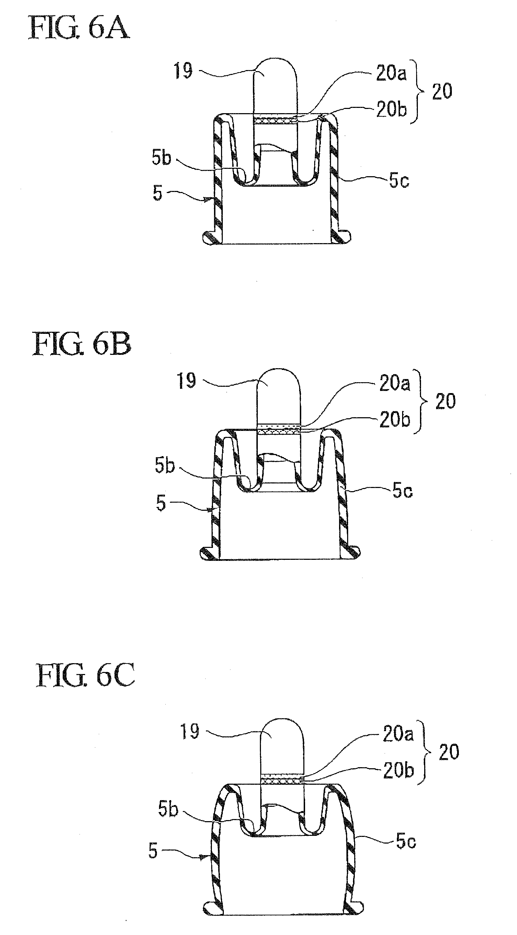

Here, the portion folded upward functions as the index body 19 in the invention. The index body 19 is formed as a cylinder with a hemispherical upper surface. The index body 19 includes a colored index, section 20 as shown in FIGS. 6A, 6B and 6C. The index section 20 indicates the internal pressure in the cuff 90 at three levels. While the index body 19 itself is white-colored, for example, the index section 20 has colored sections having different colors along the circumferential direction of the index body 19. Namely, the index section 20 has a first colored section 20a (for example, blue-colored) and a second colored section 20b (for example, red-colored) which is located further toward the opening 5a from the first colored section 20a. This configuration allows the user to determine the degree of inflation of the balloon 5, i.e., the internal pressure in the cuff 90 by visually recognizing the color(s) of the index section 20.

The bellow-shaped expandable section 5b contracts as shown in FIG. 6A when the pressure inside of the balloon 5 is below the predetermined pressure and thus the index body 19 provided in the center of the expandable section 5b is retracted with respect to the balloon 5. In this state, both of the first colored section 20a and the second colored section 20b on the index body 19 are buried in the side cylinder portion 5c of the balloon 5 and thus cannot be recognized visually from outside. Accordingly, in this state, it is determined that the internal pressure in the cuff 90 has not reached the predetermined pressure.

When the internal pressure in the balloon 5 is within a suitable range, the inflation of the balloon 5 causes the expandable section 5b to expand as shown in FIG. 6B, and thus the index body 19 moves upward so as to allow the user to visually recognize the first colored section 20a. Here, it is determined that the internal pressure in the cuff 90 is suitable. When the internal pressure in the balloon 5 becomes excessively large (i.e., becomes unusual) as shown in FIG. 6C, the user can visually recognize the second colored section 20b along with the first colored section 20a. Here, it is determined that the internal pressure in the cuff 90 is excessively large (unusual).

As shown in FIGS. 3A, 3B and 4, the cap 7 is fit into and fixed to the balloon accommodating section 8 of the main body 6. As shown in FIG. 4, the cap 7 is constituted by a pair of side walls 7a and 7a, and a cover 7b provided integrally with and between the pair of side walls 7a and 7a. The cap 7 is made of comparatively hard transparent resin such as polycarbonate. Each of the side walls 7a and 7a includes a fitting portion 21 formed as a projected rim at its lower inner portion. The fitting portion 21 engages with an engaging groove 22 formed at both sides of the main body 6.

With this configuration, the cap 7 is fixed to the main body 6 while covering the balloon 5 fixed to the balloon accommodating section 8. In this state, the fitting portions 21 of the side walls 7a and 7a are fit into and engaged with the engaging grooves 22, and the side walls 7a and 7a hold the main body 6 therebetween. The fitting portions 21 may be further firmly fixed to the engaging grooves 22 by being bonded or welded to the engaging grooves 22.

An oval opening 23 is formed in each of the side walls 7a and 7a of the cap 7. The side cylinder portion 5c of the balloon 5 is exposed through the opening 23.

This configuration allows the user to check the degree of inflation of the balloon 5 by touching and pinching the side cylinder portions 5c from both sides through the openings 23 and 23.

The cover 7b of the cap 7 includes a recess 24 formed at a position in accordance with the index body 19 of the balloon 5 as shown in FIGS. 3A and 3B. The recess 24 is formed so as not to interfere with the index body 19 when the index body 19 moves forward (upward) as the expandable section 5b of the balloon 5 expands. Since the cap 7 is made of transparent resin as described above, the index body 19 positioned in the recess 24 can readily be visually recognized. Accordingly, the color of the index section 20 after the index body 19 moved forward (upward) can readily be visually recognized from outside. The cap 7 functions as a lens due to the difference between the refractive index of the transparent resin and the refractive index of air, and thus the index body 19 can readily be visually recognized.

Next, usage of the thus-configured cuffed medical device 50 (tracheal tube) with the indicator 1 will be described.

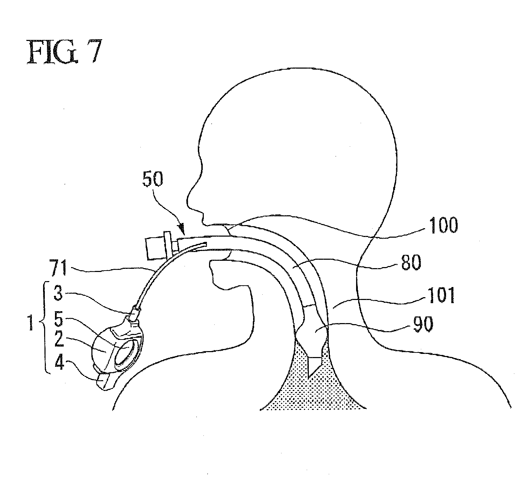

As shown in FIG. 7, the cuffed medical device 50 is first applied to a user according to an ordinary process. In particular, a distal end of the tube 80 is inserted from the mouth 100 of the patient and the cuff 90 is made to reach the trachea 101.

Next, an injector (not shown) is connected to the injector side connecting section 4 of the indicator 1. In particular, a tip of the injector is introduced into the inner hole 11a of the sleeve 11 through the opening 4a and is made to press the contact portion 12b of the valve element 12. The axis 12a of the valve element 12 is press-fit against the urging force from the expandable section 12c, the expandable section 12c deforms elastically and moves away from the tapered surface of the tapered portion 13b of the valve seat 13, and the small hole portion 13a is opened. In this state, the check valve 10 is in its opened state. Even if the expandable section. 12c is pressed against the step portion 9c, since the V-shaped cutouts 14 are formed in the expandable section 12c as described above, the communication path (through hole) 9 is not blocked by the expandable section 12c.

In this state, the injector is operated to inject a predetermined amount of air. The injected air passes through the small hole section 13a, the tapered portion 13b and the communication path 9. Then some air is introduced into the balloon 5 through the branch section 9b and the remainder of air passes through the connecting tube 71 connected to a cuff side connecting pipe 2 and through the second hole 83 of the tube 80 to flow into the cuff 90 from the first opening 84. The cuff 90 is inflated by the introduced air to the degree of inflation in accordance with the internal pressure. When the cuff is held in its inflated state, the balloon 5 communicating with the cuff 90 via the communication path 9 (including the branch section 9b) is inflated in accordance with the internal pressure in the cuff 90.

After the air is injected, the injector is removed from the injector side connecting section 4. Then, the pressing force toward the contact portion 12b is released and the valve body 12 is urged with the urging force caused by elastic restoration of the expandable section 12c. The outer surface of the bottom of the bowl-shaped expandable section 12c is again brought into airtight contact with the tapered surface of the tapered portion 13b so as to airtightly block the small hole section 13a. As a result, the check valve 10 is closed again.

When the small hole section 13a is blocked and the check valve 10 is closed, the cuff 90 is airtightly closed at the connecting tube 71 side by the indicator 1. Thus, the cuff 90 is closed from the atmosphere. Since the inside of the balloon 5 is in communication with the inside of the cuff 90, the balloon 5 inflates in accordance with the internal pressure in the cuff 90. Since the inside of the balloon 5 is in communication with the atmosphere (outside) through the opening 23, the balloon 5 is inflated from a state with insufficient air kept therein to a state with sufficient air due to difference in internal pressure in the cuff 90 and the atmospheric pressure, i.e., in accordance with the internal pressure in the cuff 90.

If the internal pressure in the cuff 90 is not in a desired range or below the predetermined (i.e., set) pressure, the balloon 5 is in its contracted state as shown in FIG. 6A. Thus, the index section 20 of the index body 19 is buried in the side cylinder section 5c of the balloon 5 and both of the first colored section 20a and the second colored section 20b cannot be recognized visually from outside. Accordingly, in this state, it is determined that the internal pressure in the cuff 90 is below the predetermined pressure. When the internal pressure in the cuff 90 is in a desired (proper) range, the balloon 5 is inflated properly as shown in FIG. 6B, and the expandable section 5b is expanded to move the index body 19 upward. In this state, only the first colored section 20a can be recognized visually and a second colored section 20b still cannot be recognized visually. Accordingly, in this state, it is determined that the internal pressure in the cuff 90 is in a suitable range.

When the internal pressure in the cuff 90 becomes excessively larger than the desired range, as shown in FIG. 6C, the user can visually recognize the second colored section 20b along with the first colored section 20a. Here, it is determined if the internal pressure in the cuff 90 is excessively large (unusual).

In this manner, the user can readily determine whether or not the internal pressure in the cuff 90 is in a suitable range by visually recognizing the index body 19 of the balloon 5 accommodated in the indicator main body 2 so as to check (visual recognition) the color of the index section 20.

Other than visual recognition, the user may detect the internal pressure in the cuff 90 by touching the side cylinder portion 5c of the balloon 5 exposed through the opening 23 with fingers and pinches from both sides. Thus, the user can determine whether or not the internal pressure in the cuff 90 is in a suitable range more reliably by checking the degree of inflation of the balloon 5 in two ways, both visually and tactually.

When the user checks the internal pressure (degree of inflation) in the cuff 90 in this manner, if the internal pressure is below the minimum acceptable pressure, or if reduction in the internal pressure in the cuff 90 over time is confirmed, the user may again connect the injector and inject air so as to inflate the cuff 90 to a predetermined internal pressure. By visually (or tactually) recognizing the degree of inflation (degree of inflation and deflation) of the balloon 5 while injecting air with the injector, the cuff 90 can be inflated suitably.

Then, artificial respiration is given to the patient with an artificial respirator (not shown) connected to the connector 60 of the cuffed medical device (tracheal tube) 50.

The indicator 1 is configured such that the balloon 5 communicating with the cuff 90 inflates (or deflates) in accordance with internal pressure in the cuff 90. Thus, the user can readily and precisely determine reduction of the internal pressure in the cuff 90 through visual recognition of the index body 19 of the balloon 5. Further, the user can detect the internal pressure in the cuff 90 by touching with fingers the balloon 5 in order to check the degree of inflation (degree of inflation and deflation). Thus, the user can determine whether or not the internal pressure in the cuff 90 is in a suitable range more reliably by checking the degree of inflation of the balloon 5 visually and tactually. With this configuration, the conventional inconvenience that a detection of reduction in internal pressure requires time and effort or requires a certain amount of experience can be avoided and any unexpected accidents can be reliably prevented.

Since the cuff side connecting section 3 is formed integrally with the indicator main body 2, the cuff side connecting section 3 cannot be removed from the indicator main body 2 during operation of the cuffed medical device 50. Thus, operability of the cuffed medical device 50 can be improved.

The expandable section 5b of the balloon 5 provided with the index body 19 is bellow-shaped. The expandable section 5b expands and contracts mechanically to move the index body 19 forward and backward. In this manner, compared with a case in which the expandable section expands and contracts due only to elastic deformation of a material made of an elastic body, the expandable section 5b suffers from less deterioration such as fatigue and its restorability is maintained over time. As a result, the relationship between the internal pressure in the cuff 90 and the forward and backward movement of the index body 19 is kept constant over time, and thus reliability in detection of the internal pressure in the cuff 90 by the index body 19 is secured over time.

It is to be understood that the invention is not limited to those embodiments described above, and various modifications may be made without departing from the sprit and scope of the invention. For example, while the cap 7 covers the balloon 5 and thus the index body 19 in the illustrated embodiment, the cover 7b of the cap 7 may have a through hole through which the index body 19 passes. That is, the recess 24 shown in FIGS. 3A and 3B may alternatively be a through hole for making the index body 19 protrudes through the cap 7. With this configuration, the thickness of the cover 7b can be reduced to provide a compact and lightweight indicator 1.

Although the cap 7 is constituted by a pair of side walls 7a and 7a and the cover 7b between the side walls, the side walls may be cylindrically shaped. With this configuration, the strength of the cap 7 itself can be increased so as to improve protective function of the balloon 5.

The index section 20 of the index body 19 is formed by two colored sections: the first colored section 20a and the second colored section 20b. The index section 20, however, may be formed only of the first colored section, or may alternatively be formed of three or more colored sections.

The configuration of the check valve and the shape of the balloon 5 are not limited to those described above. Rather, various configurations and shapes may alternatively be employed.

The cuffed medical device in which the invention is employed may also be applied to various applications including a balloon catheter for a pulmonary artery other than the described tracheal tube.

## Claims

1. An indicator for a cuffed medical device, attached to a cuffed medical device to indicate internal pressure in a cuff of the medical device, the indicator comprising:
an indicator main body which includes a check valve adapted to be connected to injection means such as an injector for injecting a fluid into the cuff, a connecting section formed integrally with the indicator main body for connection to the cuffed medical device, the connecting section communicating with the inside of the cuff, and a communication path for making the check valve and the connecting section communicate with each other; and
a balloon provided in the indicator main body,
wherein the inside of the balloon is made to communicate with the communication path and is inflatable and deflatable in accordance with the internal pressure in the cuff; and
the balloon includes an index body which is moved forward and backward in accordance with the degree of inflation and deflation of the balloon in order to make the degree of inflation and deflation of the balloon visually recognizable.

2. An indicator for a cuffed medical device according to claim 1 wherein the balloon includes a bellow-shaped expandable section in which the index body is provided, the bellow-shaped expandable section adapted to expand and contract so as to move the index body forward and backward.

3. An indicator for a cuffed medical device according to claim 1 or 2, wherein the balloon is accommodated in the indicator main body, and the indicator main body has an opening formed thereon through which an inflating/deflating surface of the balloon is exposed.
